# FASCICULE DE BREVET EUROPEEN

(11) **EP 1 781 198 B1**
(45) Date de publication et mention de la délivrance du brevet: **28.01.2015**
(21) Numéro de dépôt: 05798589.7
(22) Date de dépôt: 25.08.2005
(51) Int. Cl.: A61B 17/88, A61B 17/58

(54) **IMPLANT COMPRENANT UNE OU PLUSIEURS ELECTRODES ET INSTRUMENT DE POSE ASSOCIE**
IMPLANTAT MIT EINER ODER MEHREREN ELEKTRODEN UND DAZUGEHÖRIGES EINFÜHRUNGSINSTRUMENT
IMPLANT COMPRISING ONE OR MORE ELECTRODES AND CORRESPONDING INSERTION INSTRUMENT

(30) Priorité: 25.08.2004 FR 0409092
(43) Date de publication de la demande: 09.05.2007
(73) Titulaire: SPINEGUARD, 94160 Saint Mandé (FR)
(72) Inventeur: BOURLION, Maurice, F-42400 Saint-Chamond (FR)
(74) Mandataire: Cabinet Plasseraud
(86) Numéro de dépôt international: PCT/FR2005/002143
(87) Numéro de publication internationale: WO 2006/024801

(56) Documents cités:
- EP-A- 0 781 532
- DE-A1- 3 414 992
- FR-A- 2 835 732
- GB-A- 2 356 051
- US-A- 6 030 162
- US-A1- 2003 229 354
- US-B1- 6 402 757

## Description

La présente invention concerne le domaine de l'orthopédie générale, et plus particulièrement la pose d'implants, notamment d'implants du type vis, broches, etc., pour la mise en place de dispositifs permettant par exemple la correction et la stabilisation de la colonne vertébrale ou la réduction de fractures.

De tels implants sont destinés à être positionnés dans la structure osseuse.

Une des principales difficultés dans la pose de ces implants est, pour le praticien, de parvenir à positionner correctement ces implants.

Or, un mauvais positionnement de l'implant peut ne pas donner un résultat thérapeutique satisfaisant. Il peut également provoquer chez le patient des douleurs, paralysies, hémorragies, etc., nécessitant souvent une nouvelle intervention chirurgicale, voire dans certains cas causant des dommages irréparables.

Il s'avère donc nécessaire pour le praticien de pouvoir non seulement suivre, mais également guider, l'implant lors de sa pose.

L'art antérieur connaît déjà des dispositifs permettant le suivi de la pénétration d'un instrument dans une structure anatomique, en particulier une structure osseuse.

En particulier, on connaît de la demande de brevet FR2835732, déposée par le présent demandeur, un dispositif permettant de suivre la pénétration d'un instrument de forage dans la vertèbre par la mesure des différences d'impédance électrique au fur et à mesure de la pénétration, de sorte que le praticien sait, à chaque instant, si l'extrémité de l'instrument sort du cortex osseux et pénètre dans une zone de tissus mous (moelle, nerfs, tissus). Dans ce cas, le praticien modifie la trajectoire de l'instrument de pénétration pour revenir dans le cortex osseux. Un tel dispositif permet ainsi de détecter la formation d'une brèche dans le cortex osseux au moment du forage de l'avant-trou. Pour ce faire, ledit dispositif de suivi objet de la demande comporte au moins un électrostimulateur apte à réaliser une stimulation neuromusculaire et pouvant être relié à au moins deux électrodes dont l'une au moins est située à une extrémité distale de l'instrument de forage, au moins un impédancemètre relié à au moins deux électrodes dont l'une au moins est située à une extrémité distale de l'instrument de forage, et au moins un dispositif de signalisation capable de produire un signal en cas de détection, par l'impédancemètre d'une variation d'impédance.

Cependant, un tel dispositif ne permet pas d'effectuer le suivi de la mise en place de l'implant dans l'avant-trou.

Le document DE 44 20 232 décrit un autre dispositif permettant d'assurer le suivi de la pénétration d'un instrument dans une structure anatomique. Le document EP 0 781 532 décrit un implant selon le préambule de la revendication 1.

Le but de la présente invention est de proposer un équipement permettant le suivi et le guidage d'un implant lors de sa mise en place dans une structure osseuse, cette dernière étant forée soit directement par l'implant lors de sa pose, soit lors d'une opération préalable.

A cet effet, l'invention concerne selon son acception la plus générale un implant destiné à être placé dans une structure anatomique, en particulier une structure osseuse.

L'invention est remarquable en ce que ledit implant comprend au moins deux électrodes séparées l'une de l'autre par un isolant, lesdites électrodes affleurant la surface dudit implant de façon à permettre le suivi et le guidage dudit implant lors de sa mise en place dans la structure anatomique.

De préférence, lesdites électrodes sont disposées de telle sorte que :
i) l'une des électrodes affleure au moins en partie la surface périphérique dudit implant,
ii) l'autre électrode affleure ponctuellement la surface périphérique dudit implant.

Avantageusement, lesdites électrodes sont disposées de telle sorte que :
i) l'une des électrodes forme au moins en partie la surface périphérique dudit implant,
ii) l'autre électrode, interne audit implant, affleure ponctuellement la surface distale dudit implant.

L'implant ainsi configuré, il est possible de suivre la progression de sa pose dans la structure anatomique lorsqu'il est positionné à l'aide de l'instrument de pose adapté, lequel est décrit plus loin.

De préférence, l'électrode externe est de forme sensiblement tubulaire.

De même, ladite électrode interne est avantageusement de forme sensiblement tubulaire.

Avantageusement, lesdites électrodes sont coaxiales ou excentriques.

Dans un mode de réalisation préféré, la (ou les) électrode(s) est (sont) amovible(s).

Avantageusement, ledit isolant est également amovible. Dans ce cas, son retrait sera effectué communément avec ladite électrode interne ou après le retrait de cette dernière.

La présente invention porte également sur l'implant non pourvu de la partie amovible précédemment décrite, à savoir l'électrode interne, et éventuellement l'isolant. Dans ce cas, l'implant, traversé par un canal, comporte au moins une électrode affleurant une partie au moins de la surface périphérique dudit implant, constituant en tout ou partie la paroi latérale dudit implant.

De préférence, ledit implant est constitué par une électrode tubulaire.

Avantageusement, ledit implant comporte un isolant de forme tubulaire.

Avantageusement, ledit canal traverse longitudinalement ledit implant.

Avantageusement, le canal est un canal central.

La présente invention se rapporte également à un instrument de pose destiné à placer les implants précédemment décrits dans une structure anatomique, en particulier une structure anatomique osseuse. Pour ce faire, ledit instrument est relié à une source de tension alimentant au moins deux électrodes et à un moyen de mesure de l'impédance entre lesdites électrodes.

Dans le cas d'un implant non pourvu de la partie amovible telle que décrite précédemment, ledit instrument comporte une partie longitudinale interne. Cette partie longitudinale est destinée à se loger dans le canal de l'implant. Selon que l'implant soit pourvu ou non d'un isolant, ladite partie longitudinale sera constituée d'une électrode ou bien d'une électrode entourée d'un isolant.

Avantageusement, la partie longitudinale dudit instrument est amovible. Ainsi, sans partie longitudinale, ledit instrument est adapté pour venir en prise avec un implant comprenant lui-même une électrode interne, laquelle est séparée de l'électrode externe par un isolant.

Avantageusement, ledit instrument comporte des moyens permettant le contact électrique avec la (ou les) électrode(s) lorsque ledit implant est en prise par ledit instrument. Le contact électrique pourra être réalisé de manière interne et/ou de manière externe. Ainsi, pour un contact interne, ledit instrument de pose comportera des éléments de contact électriques positionnés pour être en contact avec l'électrode interne de l'implant ; pour un contact externe, ledit instrument comportera des éléments de contact électriques positionnés pour être en contact avec l'électrode périphérique dudit implant.

Avantageusement, ledit instrument comporte une cavité pour recevoir en partie ledit implant, ainsi que des moyens permettant de centrer ledit implant logé dans ledit instrument.

Avantageusement, ledit instrument de pose comporte des moyens d'entraînement mécanique de l'implant.

L'implant et l'instrument ainsi configuré, il est alors possible de suivre la progression de l'implant lors de sa pose dans la structure osseuse par l'analyse d'un signal électrique établi au moyen desdites électrodes.

Lorsque l'extrémité distale du système, formé de l'instrument et de l'implant, est au contact d'une brèche formée dans la structure osseuse, l'impédance décroît brutalement. Ainsi informé, le patricien rectifie la trajectoire donnée à l'implant pour venir le repositionner dans la structure osseuse. Il est procédé ainsi jusqu'à parvenir à la mise en place finale dudit implant.

On comprendra mieux l'invention à l'aide de la description, faite ci-après à titre purement explicatif, d'un premier mode de réalisation de l'invention, en référence aux figures annexées :
- la figure 1 illustre une vue en coupe d'un implant selon un mode de réalisation de l'invention ;
- la figure 2 illustre une vue en coupe de l'implant de la figure 1 après le retrait de la partie amovible dudit implant, ainsi qu'une vue en coupe de la partie amovible retirée de l'implant ;
- la figure 3 illustre une vue schématique d'un instrument de pose en prise avec l'implant de la figure 1 ; et
- la figure 4 illustre une vue en coupe d'un implant selon un second mode de réalisation de l'invention, ledit implant étant en prise avec un instrument de pose.

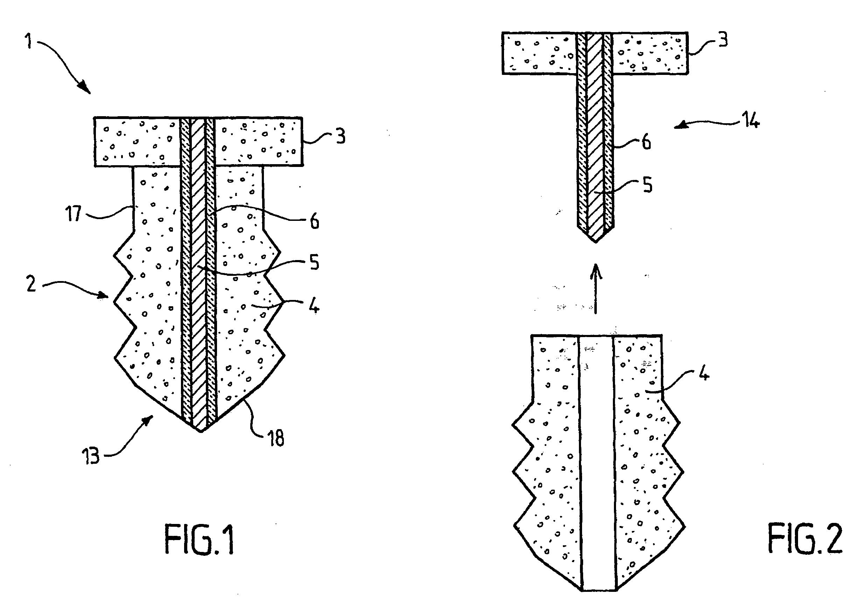

La figure 1 illustre une vue en coupe d'un implant (1) selon un premier mode de réalisation de l'invention.

Dans ce mode de réalisation, l'implant (1), du type vis pédiculaire, présente un corps (2) fileté cylindrique présentant une extrémité distale (13) en forme de pointe, l'autre extrémité étant pourvue d'une tête (3). Bien entendu, la présente invention ne se limite pas à cette configuration d'implant, celle-ci ayant été choisie dans le cas présent pour sa simplicité et donc faciliter la description de l'invention.

Le corps (2) dudit implant (1) comprend deux parties conductrices séparées par un isolant (6).

Plus particulièrement, chacune des parties conductrices est constituée par une électrode (4, 5) : une première électrode (4) formant la paroi latérale dudit implant (1) (« électrode externe ») de sorte que l'électrode externe (4) affleure la surface latérale (17) et une partie de la surface distale (18) du corps (2) dudit implant (1) ; une deuxième électrode (5) constituant une partie interne dudit implant (« électrode interne ») et affleurant ponctuellement la surface distale (18) du corps (2) dudit implant (1).

Avantageusement, l'électrode interne (5), en forme de tige de section circulaire, traverse longitudinalement ledit implant (1). Elle est entourée de l'isolant (6) lui-même entouré de l'électrode extérieure (4).

Selon un mode de réalisation préféré de l'invention, lesdites électrodes (4, 5) sont disposées de sorte à être coaxiales. Bien entendu, il s'agit d'un mode particulier de réalisation, étant entendu que le corps (2) dudit implant (1) peut être constitué par exemple de deux électrodes excentriques.

Afin de faciliter le contact électrique avec un instrument destiné à la pose d'un tel implant (2) dans une structure osseuse, l'électrode interne (5) et l'isolant (6) se prolongent au travers de la tête (3) dudit implant (1).

Selon un mode de réalisation particulièrement avantageux de l'invention, ledit implant (1) comporte une partie amovible (14). Ainsi, lorsque ladite partie amovible (14) est retirée, généralement une fois l'implant (1) positionné dans la Structure osseuse, un canal est formé dans ledit implant (1), selon son axe longitudinal (cf. figure 2).

De préférence, la partie amovible (14) comprend à la fois l'électrode interne (5) et l'isolant (6). Cependant, il est évident qu'un homme du métier pourra prévoir que la partie amovible (14) de l'implant (1) soit constituée seulement de l'électrode interne (5), l'isolant (6) restant dans ledit implant (1) lors du retrait de ladite partie amovible (14).

Le retrait de certaines parties de l'implant, telles que l'électrode interne et l'isolant dans l'exemple décrit ci-dessus, permet d'éviter de laisser implanter dans le corps du patient plusieurs matériaux.

En outre, seul les matériaux restant dans le corps du patient devant être impérativement dits « implantables », les parties de l'implant retirées une fois ce dernier disposé dans la structure osseuse peuvent n'être constituées que de matériaux biocompatibles. Les matériaux biocompatibles étant moins onéreux que les matériaux implantables, un implant comprenant une partie amovible présentera ainsi l'avantage de présenter un coût de fabrication inférieur à un implant ne comprenant pas de partie amovible.

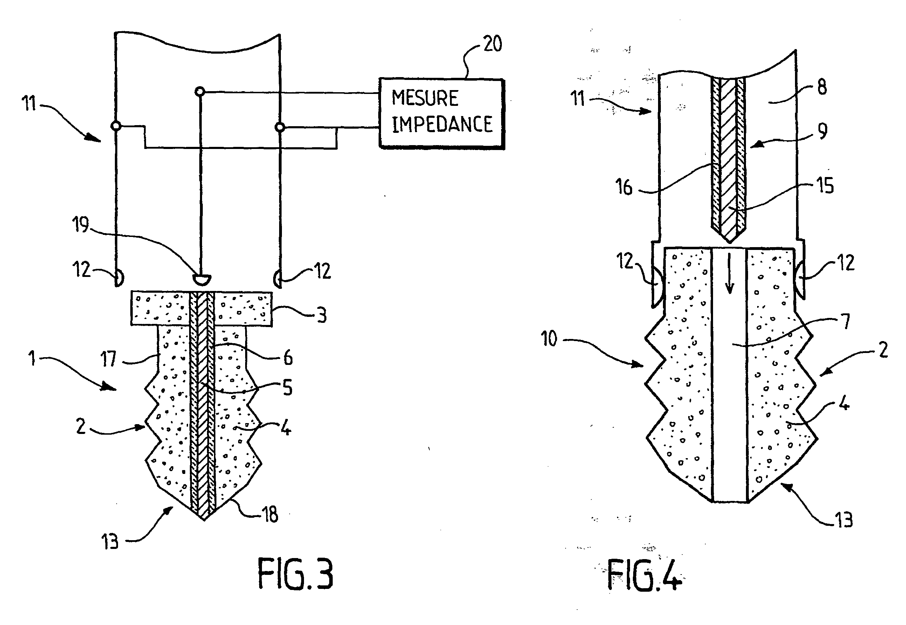

La figure 3 illustre une vue simplifiée du contact électrique entre un instrument de pose (11) d'un implant dans une structure osseuse, et l'implant (1) précédemment décrit. Afin de simplifier la figure, il a été représenté un contact non effectif entre ledit implant (1) et ledit instrument (11).

Le contact électrique entre l'instrument de pose (11) et l'implant (1) est réalisé par l'intermédiaire de pattes de connexion (12, 19) constituant ledit instrument de pose (11) : une patte de connexion (19) interne venant en contact avec l'électrode (5) interne dudit implant (1), et au moins une patte de connexion (12) externe venant au contact de l'électrode externe (4) dudit l'implant (1).

En outre, s'agissant de suivre et de guider l'implant (1) lors de sa pose dans la structure osseuse, les pattes de connexion (12, 19) de l'instrument de pose (11) sont reliées à un moyen de mesure de l'impédance entre lesdites électrodes (4, 5) (impédancemètre 20).

Ledit impédancemètre (20) pourra avantageusement être relié à un dispositif d'alerte (non représenté) pouvant émettre un signal d'alerte visuel, sonore et/ou tactile lors d'une variation de l'impédance mesurée ou en cas de franchissement d'un seuil d'impédance.

De même, ledit impédancemètre (20) pourra avantageusement être relié à un dispositif de visualisation (non représenté) permettant de visualiser les variations de l'impédance mesurée entre lesdites électrodes (4, 5) par l'impédancemètre (20). Il pourra s'agir par exemple d'un écran de visualisation permettant le suivi, sous forme de courbes, de la variation de l'impédance au fur et à mesure de la pénétration de l'implant (1) dans la structure osseuse.

La figure 4 illustre une vue en coupe d'un implant (10) selon un autre mode de réalisation de l'invention et d'un instrument de pose (11) destiné à placer ledit implant (10) dans la structure osseuse.

Afin de faciliter la compréhension du principe de coopération de l'implant (10) avec ledit instrument (11), et en particulier de distinguer les éléments constituant respectivement ledit instrument de pose (11) et ledit implant (10), il a été représenté sur cette figure une coopération non effective entre ledit implant (10) et ledit instrument (11).

Dans cet exemple de réalisation, ledit implant (10) est constitué d'un corps (2) fileté conducteur présentant une extrémité distale (13) en pointe.

Avantageusement, le corps (2) dudit implant (10), de forme tubulaire, est muni d'un canal (7) traversant d'une extrémité à l'autre le corps (2) dudit implant (1).

Avantageusement, le corps (2) dudit implant (10) est constitué par une électrode (4).

Ledit instrument de pose (11) destiné à venir en prise avec ledit implant (10) est constitué d'un corps creux (8) muni d'une partie longitudinale (9) centrale, laquelle est destinée à se loger dans le canal (7) dudit implant (10) lorsque celui-ci est en prise avec ledit instrument (11).

Avantageusement, ladite partie longitudinale (9) centrale est constituée d'une électrode (15) entourée d'un isolant (16). Il est bien entendu évident que la partie longitudinale (9) pourra n'être constituée que de l'électrode (15), l'implant destiné à venir en prise avec ledit instrument de pose (11) comportant lui-même un isolant.

Ainsi, lors de sa pose dans la structure osseuse, ledit implant (10) est fixé à l'extrémité dudit instrument (11), et la partie longitudinale (9) dudit instrument (11) logée dans le canal (7) dudit implant (10).

Afin de suivre la progression de la pose de l'implant (10), une continuité électrique est établie.

Avantageusement, le contact électrique est réalisé au moyen de pattes de connexion (12) venant au contact de l'électrode (4) constituant ledit implant (10).

Il a été présenté de l'instrument de pose (11) seulement la partie électrique permettant le suivi et le guidage d'un implant dans une structure osseuse. Il est bien entendu évident pour un homme du métier que ledit instrument de pose (11) pourra comporter des moyens d'entraînement mécanique manuel ou du type moteur électrique, permettant par exemple d'entraîner l'implant en rotation, en poussée, ou autre.

L'invention est décrite dans ce qui précède à titre d'exemple. Il est entendu que l'homme du métier est à même de réaliser différentes variantes de l'invention sans pour autant sortir du cadre du brevet.

## Revendications

1. Implant (1 ; 10) destiné à être placé dans une structure anatomique, en particulier une structure osseuse, l'implant comprenant :
- au moins deux électrodes (4, 5 ; 4, 15) séparées par un isolant (6 ; 16), lesdites électrodes affleurant la surface dudit implant (1 ; 10) de façon à permettre le suivi et le guidage dudit implant (1 ; 10) lors de sa mise en place dans la structure anatomique, et
- un canal (7) qui traverse ledit implant (1 ; 10) longitudinalement,
lesdites électrodes (4, 5 ; 4, 15) étant disposées de telle sorte que :
i) l'une des électrodes (4), externe, forme au moins en partie la surface périphérique dudit implant (1 ; 10),
ii) l'autre électrode (5; 15), interne, est logée dans le canal (7) et affleure ponctuellement la surface périphérique dudit implant (1 ; 10),
l'implant (1 ; 10) étant **caractérisé en ce que** l'électrode interne (5; 15) est amovible par rapport au canal (7), l'électrode interne appartenant à l'une des parties choisie parmi une partie amovible (14) de l'implant (1) et une partie longitudinale (9) interne d'un instrument de pose (11) destiné à placer l'implant (1 ; 10) dans la structure anatomique.

2. Implant (1 ; 10) selon la revendication 1, **caractérisé en ce que** l'électrode interne (5 ; 15) affleure ponctuellement la surface distale dudit implant (1 ; 10).

3. Implant (1 ; 10) selon la revendication 1 ou 2, **caractérisé en ce que** l'électrode (4) externe est de forme sensiblement tubulaire.

4. Implant (1 ; 10) selon l'une quelconque des revendications 1 à 3, **caractérisé en ce que** l'électrode (5 ; 15) interne est de forme sensiblement tubulaire.

5. Implant (1 ; 10) selon l'une quelconque des revendications précédentes, **caractérisé en ce que** lesdites électrodes (4, 5 ; 4, 15) sont coaxiales.

6. Implant (1 ; 10) selon l'une quelconque des revendications 1 à 4, **caractérisé en ce que** lesdites électrodes (4, 5 ; 4, 15) sont excentriques.

7. Implant (1 ; 10) selon l'une quelconque des revendications précédentes, **caractérisé en ce que** l'électrode externe est amovible.

8. Implant (1; 10) selon l'une quelconque des revendications précédentes, **caractérisé en ce que** ledit isolant (6 ; 16) est amovible.

9. Implant (1 ; 10) selon l'une quelconque des revendications 1 à 8, **caractérisé en ce que** l'isolant (6 ; 16) est de forme tubulaire.

10. Implant (1 ; 10) selon l'une quelconque des revendications 1 à 9, **caractérisé en ce que** ledit canal (7) est central.

11. Instrument de pose (11) destiné à placer dans une structure anatomique, en particulier une structure anatomique osseuse, un implant (1 ; 10) selon l'une quelconque des revendications 1 à 10, ledit instrument de pose (11) étant relié à une source de tension alimentant lesdites au moins deux électrodes de l'implant (1 ; 10) et à un moyen de mesure de l'impédance entre lesdites électrodes, l'instrument de pose (11) comportant des moyens permettant le contact électrique avec les électrodes (4, 5 ; 4, 15) lorsque ledit implant (1 ; 10) est en prise par ledit instrument de pose (11), de façon à permettre le suivi et le guidage dudit implant (1 ; 10) lors de sa mise en place dans la structure anatomique.

12. Instrument de pose (11) selon la revendication 11, **caractérisé en ce qu'**il comporte la partie longitudinale (9) interne destinée à se loger dans le canal (7) dudit implant (10), ladite partie longitudinale (9) étant constituée par une électrode (15).

13. Instrument de pose (11) selon la revendication 11, **caractérisé en ce que** ladite électrode (15) de la partie longitudinale (9) est entourée d'un isolant (16).

14. Instrument de pose (11) selon l'une quelconque des revendications 12 et 13, **caractérisé en ce que** la partie longitudinale (9) dudit instrument de pose (11) est amovible par rapport audit instrument de pose (11).

15. Instrument de pose (11) selon l'une quelconque des revendications 11 à 14, **caractérisé en ce qu'**il comporte en outre une cavité pour recevoir en partie ledit implant (1 ; 10), ainsi que des moyens permettant de centrer ledit implant (1 ; 10) logé dans ledit instrument (11).

16. Instrument de pose (11) selon l'une quelconque des revendications 11 à 15, **caractérisé en ce qu'**il comporte des moyens d'entraînement mécanique de l'implant (1, 10).

17. Ensemble comprenant un implant selon l'une quelconque des revendications 1 à 10 et un instrument de pose selon l'une quelconque des revendications 11 à 16.

## Patentansprüche

1. Implantat (1; 10), welches dazu bestimmt ist, in einer anatomischen Struktur, insbesondere einer Knochen-Struktur, positioniert zu werden, wobei das Implantat umfasst:
- wenigstens zwei durch einen Isolator (6; 16) getrennte Elektroden (4, 5; 4, 15), wobei die Elektroden mit der Oberfläche des Implantats (1; 10) fluchten, so dass die Verfolgung und die Führung des Implantats (1; 10) bei seiner Positionierung in der anatomischen Struktur ermöglicht wird, und
- einen Kanal (7), welcher das Implantat (1; 10) longitudinal durchquert,
wobei die Elektroden (4, 5; 4, 15) so angeordnet sind, dass:
i) die eine, externe, der Elektroden (4) wenigstens teilweise die Umfangsfläche des Implantats (1; 10) bildet
ii) die andere, interne Elektrode (5; 15) in dem Kanal (7) aufgenommen ist und punktuell mit der Umfangsfläche des Implantats (1; 10) fluchtet, wobei das Implantat (1; 10) **dadurch gekennzeichnet ist, dass** die interne Elektrode (5; 15) in Bezug auf den Kanal (7) abnehmbar ist, wobei die interne Elektrode zu einem von Teilen gehört, die ausgewählt sind aus einem abnehmbaren Teil (14) des Implantats (1) und einem longitudinalen internen Teil (9) eines Positionierungsinstruments (11), das dazu bestimmt ist, das Implantat (1; 10) in der anatomischen Struktur zu positionieren.

2. Implantat (1; 10) nach Anspruch 1, **dadurch gekennzeichnet, dass** die interne Elektrode (5; 15) mit der distalen Fläche des Implantats (1; 10) punktuell fluchtet.

3. Implantat (1; 10) nach Anspruch 1 oder 2, **dadurch gekennzeichnet, dass** die externe Elektrode (4) im Wesentlichen rohrförmig ist.

4. Implantat (1; 10) nach einem der Ansprüche 1 bis 3, **dadurch gekennzeichnet, dass** die interne Elektrode (5; 15) im Wesentlichen rohrförmig ist.

5. Implantat (1; 10) nach einem der vorhergehenden Ansprüche, **dadurch gekennzeichnet, dass** die Elektroden (4, 5; 4, 15) koaxial sind.

6. Implantat (1; 10) nach einem der Ansprüche 1 bis 4, **dadurch gekennzeichnet, dass** die Elektroden (4, 5; 4, 15) exzentrisch sind.

7. Implantat (1; 10) nach einem der vorhergehenden Ansprüche, **dadurch gekennzeichnet, dass** die externe Elektrode abnehmbar ist.

8. Implantat (1; 10) nach einem der vorhergehenden Ansprüche, **dadurch gekennzeichnet, dass** der Isolator (6; 16) abnehmbar ist.

9. Implantat (1; 10) nach einem der Ansprüche 1 bis 8, **dadurch gekennzeichnet, dass** der Isolator (6; 16) rohrförmig ist.

10. Implantat (1; 10) nach einem der Ansprüche 1 bis 9, **dadurch gekennzeichnet, dass** der Kanal (7) zentral ist.

11. Positionierungsinstrument (11), welches dazu bestimmt ist, ein Implantat (1; 10) nach einem der Ansprüche 1 bis 10 in einer anatomischen Struktur, insbesondere einer Knochen-Struktur, zu positionieren, wobei das Positionierungsinstrument (11) mit einer Spannungsquelle verbunden ist, welche die wenigstens zwei Elektroden des Implantats (1; 10) versorgt, und mit einem Messmittel der Impedanz zwischen den Elektroden, wobei das Positionierungsinstrument (11) Mittel umfasst, welche den elektrischen Kontakt mit den Elektroden (4, 5; 4, 15) ermöglichen, wenn das Implantat (1; 10) von dem Positionierungsinstrument (11) ergriffen wird, so dass die Verfolgung und die Führung des Implantats (1; 10) bei seiner Positionierung in der anatomischen Struktur ermöglicht wird.

12. Positionierungsinstrument (11) nach Anspruch 11, **dadurch gekennzeichnet, dass** es den internen longitudinalen Teil (9) umfasst, der dazu bestimmt ist, in dem Kanal (7) des Implantats (10) aufgenommen zu sein, wobei der longitudinale Teil (9) durch eine Elektrode (15) gebildet wird.

13. Positionierungsinstrument (11) nach Anspruch 11, **dadurch gekennzeichnet, dass** die Elektrode (15) des longitudinalen Teils (9) von einem Isolator (16) umgeben ist.

14. Positionierungsinstrument (11) nach einem der Ansprüche 12 und 13, **dadurch gekennzeichnet, dass** der longitudinale Teil (9) des Positionierungsinstruments (11) in Bezug auf das Positionierungsinstrument (11) abnehmbar ist.

15. Positionierungsinstrument (11) nach einem der Ansprüche 11 bis 14, **dadurch gekennzeichnet, dass** es weiter eine Kavität umfasst, um teilweise das Implantat (1; 10) aufzunehmen, sowie Mittel, die es ermöglichen, das in dem Instrument (11) aufgenommene Implantat (1; 10) zu zentrieren.

16. Positionierungsinstrument (11) nach einem der Ansprüche 11 bis 15, **dadurch gekennzeichnet, dass** es mechanische Antriebsmittel für das Implantat (1; 10) umfasst.

17. Anordnung, umfassend ein Implantat nach einem der Ansprüche 1 bis 10 und ein Positionierungsinstrument nach einem der Ansprüche 11 bis 16.

## Claims

1. Implant (1; 10) intended for being placed in an anatomical structure, in particular a bone structure, the implant comprising:
- at least two electrodes (4, 5; 4, 15) separated by an insulator (6; 16), said electrodes being flush with the surface of said implant (1; 10) so as to allow said implant (1; 10) to be followed up and guided during its insertion in the anatomical structure, and
- a channel (7) that passes through said implant (1; 10) longitudinally,
said electrodes (4, 5; 4, 15) being arranged so that:
i) one (4) of the electrodes, external, at least partially forms the peripheral surface of said implant (1; 10),
ii) the other electrode (5), internal, is housed within the channel (7) and is punctually flush with the peripheral surface of said implant (1; 10),
the implant being **characterised in that** the internal electrode (5; 15) is removable with respect to the channel (7), the internal electrode belonging to one of the parts chosen between a removable part (14) of the implant (1) and an internal longitudinal part (9) of an insertion instrument (11) intended for placing the implant in the anatomical structure.

2. Implant (1; 10) according to claim 1, **characterised in that** the internal electrode (5; 15) is selectively flush with the distal surface of said implant (1; 10).

3. Implant (1; 10) according to claim 1 or 2, **characterised in that** the external electrode (4) is substantially tubular.

4. Implant (1; 10) according to any of claims 1 to 3, **characterised in that** the internal electrode (5; 15) is substantially tubular.

5. Implant (1; 10) according to any one of the preceding claims, **characterised in that** the electrodes (4, 5; 4, 15) are coaxial.

6. Implant (1; 10) according to any one of the claims 1 to 4, **characterised in that** the electrodes (4, 5; 4, 15) are eccentric.

7. Implant (1; 10) according to any one of the preceding claims, **characterised in that** the external electrode is removable.

8. Implant (1; 10) according to any one of the preceding claims, **characterised in that** said insulator (6; 16) is removable.

9. Implant (1; 10) according to any one of claims 1 to 8, **characterised in that** the insulator (6; 16) is tubular.

10. Implant (1; 10) according to any one of claims 1 to 9, **characterised in that** said channel (7) is central.

11. Insertion instrument (11) intended for inserting in an anatomical structure, in particular a anatomical bone structure, an implant (1; 10) according to any of claims 1 to 10, said insertion instrument (11) being connected to a power source supplying said at least two electrodes of the implant (1; 10) and to a means for measuring the impedance between said electrodes, said insertion instrument (11) comprising means allowing electrical contact with said electrodes (4, 5; 4, 15) when said implant (1; 10) is in contact with said insertion instrument (11), so as to allow said implant (1; 10) to be followed up and guided during its insertion in the anatomical structure.

12. Insertion instrument (11) according to claim 11, **characterised in that** it comprises the internal longitudinal part (9) intended to be housed in the channel (7) of said implant (10), said longitudinal part (9) being constituted by an electrode (15).

13. Insertion instrument (11) according to claim 11, **characterised in that** said electrode (15) of the longitudinal part (9) is surrounded by an insulator (16).

14. Insertion instrument (11) according to any of claims 12 and 13, **characterised in that** the longitudinal part (9) of said insertion instrument (11) is removable with respect said insertion instrument (11).

15. Insertion instrument (11) according to any of claims 11 to 14, **characterised in that** it further comprises a cavity for receiving part of said implant (1; 10), as well as means that allow said implant (1; 10) housed in said insertion instrument (11) to be centred.

16. Insertion instrument (11) according to any of claims 11 to 15, **characterised in that** it comprises means for mechanically driving the implant (1; 10).

17. Assembly comprising an implant according to any of claims 1 to 10 and an insertion instrument according to any of claims 11 to 16.
